# FASCICULE DE BREVET EUROPEEN

(11) **EP 2 866 838 B1**
(45) Date de publication et mention de la délivrance du brevet: **21.09.2016**
(21) Numéro de dépôt: 13745452.6
(22) Date de dépôt: 05.07.2013
(51) Int. Cl.: A61K 47/44, A61K 47/36, A61K 31/351, A61P 35/00, A61K 9/107, A61K 31/704, A61K 9/00

(54) **EMULSION ANTI-TUMORALE A BASE DE LIPIODOL POUR LE TRAITEMENT DU CANCER**
ANTITUMOR-EMULSION AUF BASIS VON LIPIODOL ZUR KREBSBEHANDLUNG
LIPIODOL-BASED ANTI-TUMOUR EMULSION FOR TREATING CANCER

(30) Priorité: 06.07.2012 FR 1256556
(43) Date de publication de la demande: 06.05.2015
(73) Titulaire: CHU DE DIJON, 21079 Dijon Cedex (FR); Université de Bourgogne, 21078 Dijon Cedex (FR)
(72) Inventeur: GUIU, Boris, F-21121 Daix (FR); BOULIN, Mathieu, F-21000 Dijon (FR); CERCUEIL, Jean-Pierre, F-21000 Dijon (FR)
(74) Mandataire: Oudin, Stéphane
(86) Numéro de dépôt international: PCT/FR2013/051616
(87) Numéro de publication internationale: WO 2014/006349

(56) Documents cités:
- WO-A1-2004/075904
- WO-A1-2012/004007
- FAVOULET PATRICK ET AL: "Increased cytotoxicity and stability of Lipiodol-pirarubicin emulsion compared to classical doxorubicin-Lipiodol: Potential advantage for chemoembolization of unresectable hepatocellular carcinoma", ANTI-CANCER DRUGS, vol. 12, no. 10, novembre 2001 (2001-11), pages 801-806, XP009164708, ISSN: 0959-4973
- YAMASAKI TAKAHIRO ET AL: "Effect of transcatheter arterial infusion chemotherapy using iodized oil and degradable starch microspheres for hepatocellular carcinoma", JOURNAL OF GASTROENTEROLOGY, vol. 47, no. 6, juin 2012 (2012-06), pages 715-722, XP002687270,
- NAGAMITSU A ET AL: "Targeted cancer chemotherapy for VX2 tumour implanted in the colon with lipiodol as a carrier", EUROPEAN JOURNAL OF CANCER, PERGAMON PRESS, OXFORD, GB, vol. 34, no. 11, 1 octobre 1998 (1998-10-01), pages 1764-1769, XP004285128, ISSN: 0959-8049, DOI: 10.1016/S0959-8049(98)00153-1

## Description

### Domaine technique

La présente invention concerne une composition pharmaceutique comprenant du Lipiodol et une molécule ayant une activité anti-tumorale et accessoirement un hydroxyethylamidon. La présente invention concerne également l'utilisation des compositions selon l'invention pour le traitement du cancer.

### Technique antérieure

Les techniques de chimio-embolisation sont employées couramment dans le domaine du traitement du cancer et plus particulièrement dans le traitement du cancer du foie.

Cette technique consiste à restreindre la circulation sanguine au niveau des tumeurs afin d'entrainer leur nécrose. Dans le cadre du traitement de la tumeur hépatique, cette procédure consiste à accéder à l'artère hépatique et plus particulièrement à la branche de l'artère hépatique alimentant la tumeur pour y injecter un agent de chimio-embolisation qui va venir obstruer les vaisseaux alimentant la tumeur.

Le Lipiodol est un des agents de chimio-embolisation couramment utilisés. Cet agent est constitué des esters éthyliques des acides gras iodés de l'huile d'oeillette. Il contient de 43 à 53% d'iode. Il est préparé par saponification de l'huile d'oeillette ce qui libère les acides gras sous forme de savons qui sont ultérieurement iodés par le chlorure d'iode et enfin estérifiés par l'éthanol.

L'huile d'oeillette est extraite des graines de pavot noir (Papaver somniferum). Les principaux acides gras compris dans cette huile sont l'acide linoléique et l'acide linolénique.

Le Lipiodol est également utilisé comme agent de contraste dans le cadre d'investigations radiologiques.

Le Lipiodol est parfois utilisé en combinaison avec une molécule ayant une activité anti-tumorale. Classiquement, la molécule ayant une activité anti-tumorale (e.g. de la Doxorubicine) est émulsionnée avec le Lipiodol.

Toutefois ces émulsions sont peu stables *in vitro* et *in vivo* et ne permettent pas d'obtenir des effets thérapeutiques optimums.

### Exposé de l'invention

La présente invention concerne notamment des compositions pharmaceutiques contenant du Lipiodol et un agent ayant une activité anti-tumorale dont la stabilité est supérieure à celle des compositions de l'art antérieur.

La présente invention concerne donc notamment une composition pharmaceutique comprenant du Lipiodol, une molécule ayant une activité anti-tumorale et un hydroxyethylamidon.

La présente invention concerne également une composition pharmaceutique comprenant du Lipiodol et de l'Idarubicine.

En effet, il a été constaté que le mélange Idarubicine/Lipiodol permet d'obtenir une émulsion stable comparée aux autres émulsions Lipiodol/molécule ayant une activité anti-tumorale. Par ailleurs, cette composition particulière présente une pharmacocinétique particulièrement avantageuse.

Dans le cadre de la présente invention, le terme « Lipiodol » entend désigner tous les esters éthyliques des acides gras iodés de l'huile d'oeillette avec une teneur en iode comprise entre 33 et 53% (en gramme d'iode par 100 mL de produit final).

Selon un mode de réalisation préféré, le terme « Lipiodol » entend désigner les produits commercialisés sous le nom de Lipiodol Ultra-Fluide® (Laboratoire Guerbet, France) et sous le nom Ethiodol® par la société Savage (Melville, NY).

Selon un mode de réalisation préféré, ladite composition pharmaceutique est remarquable en ce que ladite molécule ayant une activité anti-tumorale est choisie dans le groupe comprenant les antimétabolites, les agents alkylants, les inhibiteurs de topoisomérases, les antibiotiques anti-tumoraux et les poisons du fuseau mitotique.

Selon un mode de réalisation encore plus préféré ladite molécule-anti-tumorale est un agent intercalant, inhibiteur de topoisomérases.

Selon un mode de réalisation tout à fait préféré ledit agent intercalant, inhibiteur de topoisomérases est une anthracycline et encore plus préférentiellement l'Idarubicine.

Les déposants ont pu mettre en évidence de manière surprenante que l'Idarubicine était particulièrement efficace dans les compositions pharmaceutiques selon l'invention. En effet, cette drogue a été démontrée comme étant la plus efficace parmi les agents anticancéreux *in* vitro sur des lignées cellulaires de CHC : HepG2, SNU-398 et SNU-449.

L'homme du métier connaît l'hydroxyethylamidon qui est couramment utilisé comme solutés de remplissage vasculaire succédanés du plasma.

Selon un mode de réalisation préféré ledit hydroxyethylamidon a un poids moléculaire compris entre 100 000 et 250 000 daltons et encore plus préférentiellement entre 120 000 et 140 000 daltons.

Selon un autre mode de réalisation préféré ledit hydroxyethylamidon a un taux de substitution molaire compris entre 0.30 et 0.70 et encore plus préférentiellement entre 0.35 et 0.45.

Selon un mode de réalisation tout à fait préféré ladite composition pharmaceutique comprend uniquement du Lipiodol, une molécule ayant une activité anti-tumorale, un hydroxyethylamidon et un solvant pharmaceuticalement acceptable.

Selon un mode de réalisation tout à fait préféré ladite composition pharmaceutique comprend uniquement du Lipiodol et de l'Idarubicine et un solvant pharmaceuticalement acceptable.

Parmi les solvants pharmaceuticalement acceptables (au sens de la Pharmacopée Européenne), l'eau pour préparations injectables et le sérum physiologique sont particulièrement préférés.

Selon un mode de réalisation préféré, la quantité de ladite molécule ayant une activité anti-tumorale est comprise entre 0.002% et 0.4% masse/volume (par rapport donc au volume total de la préparation [1% m/v = 1000 mg/100 ml]). Selon un mode de réalisation encore plus préféré, la quantité de ladite molécule ayant une activité anti-tumorale est comprise entre 0.004% et 0.2% masse/volume (par rapport donc au volume total de la préparation). Selon un mode de réalisation tout à fait préféré, la quantité de ladite molécule ayant une activité anti-tumorale est comprise entre 0.03% et 0.07% masse/volume (par rapport donc au volume total de la préparation).

Selon un mode de réalisation préféré, la quantité d'hydroxyethylamidon est comprise entre 0.19% et 4.5% masse/volume (par rapport donc au volume total de la préparation [1% m/v = 1000 mg/100 ml]). Selon un mode de réalisation encore plus préféré, la quantité d'hydroxyethylamidon est comprise entre 0.38% et 2.25% masse/volume (par rapport donc au volume total de la préparation). Selon un mode de réalisation tout à fait préféré, la quantité d'hydroxyethylamidon est comprise entre 0.5% et 1.5% masse/volume (par rapport donc au volume total de la préparation).

Selon un mode de réalisation préféré, la quantité de Lipiodol est comprise entre 40 et 80% masse/volume (par rapport donc au volume total de la préparation [1% m/v = 1000 mg/100 ml]). Selon un mode de réalisation encore plus préféré, la quantité de Lipiodol est comprise entre 50% et 70% masse/volume (par rapport donc au volume total de la préparation). Selon un mode de réalisation tout à fait préféré, la quantité de Lipiodol est comprise entre 62% et 68% masse/volume (par rapport donc au volume total de la préparation).

Selon un mode de réalisation tout à fait préféré, la composition pharmaceutique selon l'invention comprend entre 0.004% et 0.2% en masse d'Idarubicine, entre 0.38% et 2.25% en masse d'hydroxyethylamidon, entre 50% et 70% en masse de Lipiodol et une quantité d'eau pour préparations injectables (voire de sérum physiologique) q.s.p 100%.

La présente invention concerne également l'utilisation d'une composition pharmaceutique selon l'invention pour la préparation d'un médicament.

La présente invention concerne une composition pharmaceutique selon l'invention pour l'utilisation pour le traitement du cancer.

La présente invention concerne également une composition pharmaceutique selon l'invention pour l'utilisation pour le traitement du cancer primitif du foie.

La présente invention concerne également un procédé de préparation d'une composition selon l'invention comprenant une étape consistant à mettre en contact du Lipiodol, une molécule ayant une activité anti-tumorale et un hydroxyethylamidon.

La présente invention concerne également un procédé de préparation d'une composition selon l'invention comprenant une étape consistant à mettre en contact du Lipiodol et de l'Idarubicine.

### Description des modes de réalisation

### Exemple 1 :

La molécule ayant une activité anti-tumorale (Idarubicine, Zavedos®, Pfizer) est préparée pour obtention d'une solution de 5ml (2 mg/ml en ajoutant simplement de l'eau pour préparations injectable dans la poudre d'Idarubicine), mise dans une seringue de 30ml.

Trois ml d'hydroxyéthylamidon 130 000 (0.06 g/ml Voluven®, Fresenius Kabi) sont ajoutés dans cette seringue, pour un total de 8ml.

Dix ml de Lipiodol (Lipiodol Ultra Fluide®, Guerbet) sont aspirés dans une autre seringue de 20ml.

Puis le mélange est réalisé en faisant passer le contenu de la seringue contenant Idarubicine + Voluven rapidement dans la seringue de Lipiodol en utilisant un robinet 3 voies, afin d'obtenir une émulsion de type « eau dans l'huile » (la chimiothérapie représente la partie aqueuse, le Lipiodol représente la partie huileuse). Ensuite une quinzaine de passages successifs sont réalisés d'une seringue à l'autre toujours via le robinet 3 voies.

Le produit est alors prêt à être injecté par voie intra-artérielle hépatique pour le traitement du cancer primitif du foie.

Les résultats expérimentaux obtenus montrent une meilleure stabilité de l'émulsion en utilisant cette dose d'hydroxyethylamidon, c'est-à-dire que l'émulsion reste stable dans le temps et ne se déphase pas ou très peu.

Par ailleurs, on peut également obtenir avec ce type d'émulsion une augmentation de la fluidité ce qui représente un avantage pour le passage dans les microcathéters.

L'injection intra-artérielle de cette émulsion donne moins de désaturation chez les patients, témoignant d'un moindre passage pulmonaire du produit. Enfin, les scanners réalisés après le traitement montre une meilleure imprégnation lipiodolée du foie et des nodules tumoraux.

### Exemple 2 :

La composition a été préparée en mélangeant un volume égal de Lipiodol et une solution à 1mg/ml d'Idarubicine. Ledit mélange a été effectué selon le protocole indiqué précédemment. La stabilité de la composition obtenue a été mesurée après incubation à 37°C pendant 30 min.. A ce stade, on observe que 95% de la composition est encore sous la forme d'une émulsion. Les micelles d'Idarubicine/Lipiodol ont une taille comprise entre 20 et 100µm.

Ces résultats sont à comparer avec ceux obtenus par Favoulet et al. (Anticancer Drugs. 2001 Nov; 12(10):801-6) montrant que les émulsions Lipiodol/Doxorubicine sont totalement déphasées 20 min. après leur préparation.

Trois patients ont été traités avec une émulsion Lipiodol (10ml) + Idarubicine (10mg). Le passage systémique d'Idarubicine a été analysé. Les résultats obtenus sont les suivants :
- Cmax Idarubicine = 12,5 +/- 4,8 ng/ml
- AUC Idarubicine = 61,6 +/- 23,7 ng.h/ml

Ces résultats peuvent être comparés aux données publiées avec l'émulsion Lipiodol/Doxorubicine (Varela et al., J Hepatol. 2007 Mar;46(3):474-81.). Cette dernière étude montrait un Cmax de 896ng/ml, sachant que la dose de Doxorubicine injectée était de 70 mg. On trouve donc 10 fois moins d'Idarubicine dans le sang veineux (12,5ng/ml) chez les patients Lipiodol-Idarubicine. Cela prouve indubitablement que la composition selon l'invention émulsion permet d'améliorer considérablement la pharmacocinétique de l'Idarubicine.

## Revendications

1. Composition pharmaceutique comprenant du Lipiodol, une molécule ayant une activité anti-tumorale et un hydroxyethylamidon.

2. Composition pharmaceutique comprenant du Lipiodol et de l'Idarubicine.

3. omposition pharmaceutique selon la revendication 1 **caractérisée en ce que** ladite molécule ayant une activité anti-tumorale est choisie dans le groupe comprenant les antimétabolites, les agents alkylants, les inhibiteurs de topoisomérases, les agents intercalants, les antibiotiques anti-tumoraux et les poisons du fuseau mitotique.

4. Composition pharmaceutique selon la revendication 3 **caractérisée en ce que** ladite molécule-anti-tumorale est un agent intercalant, inhibiteur de topoisomérases.

5. Composition pharmaceutique selon la revendication 4 **caractérisée en ce que** ledit agent intercalant, inhibiteur de topoisomérases est une anthracycline.

6. Composition pharmaceutique selon la revendication 5 **caractérisée en ce que** ladite anthracycline est l'Idarubicine.

7. Composition pharmaceutique selon la revendication 1 **caractérisée en ce que** ledit hydroxyethylamidon a un poids moléculaire compris entre 100 000 et 250 000 daltons.

8. Composition pharmaceutique selon la revendication 7 **caractérisée en ce que** ledit hydroxyethylamidon a un poids moléculaire compris entre 120 000 et 140 000 daltons.

9. Composition pharmaceutique selon la revendication 1 **caractérisée en ce que** ledit hydroxyethylamidon a un taux de substitution molaire compris entre 0.30 et 0.70.

10. Composition pharmaceutique selon la revendication 9 **caractérisée en ce que** ledit hydroxyethylamidon a un taux de substitution molaire compris entre 0.35 et 0.45.

11. Composition pharmaceutique selon la revendication 1 ou 2 **caractérisée en ce que** ledit Lipiodol est du Lipiodol Ultra-fluide.

12. Composition pharmaceutique selon l'une quelconque des revendications 1 ou 3 à 11 **caractérisée en ce que** la quantité de ladite molécule ayant une activité anti-tumorale est comprise entre 0.004% et 0.2% (masse/volume) par rapport au volume total de la préparation.

13. Composition pharmaceutique selon la revendication 2 **caractérisée en ce que** la quantité d'Idarubicine est comprise entre 0.004% et 0.2% (masse/volume) par rapport au volume total de la préparation.

14. Composition pharmaceutique selon l'une quelconque des revendications 1 ou 3 à 12 **caractérisée en ce que** la quantité d'hydroxyethylamidon est comprise entre 0.38% et 2.25% (masse/volume) par rapport au volume total de la préparation.

15. Composition pharmaceutique selon l'une des revendications précédentes **caractérisée en ce que** la quantité de Lipiodol est comprise entre 50% et 70% (masse/volume) par rapport au volume total de la préparation.

16. Composition pharmaceutique selon l'une des revendications 1 à 15 pour l'utilisation pour le traitement du cancer.

17. Composition pharmaceutique selon l'une des revendications 1 à 15 pour l'utilisation pour le traitement du cancer primitif du foie.

18. Procédé de préparation d'une composition selon l'une quelconque des revendications 1, 3 à 12 ou 14 **caractérisé en ce qu'**il comprend une étape consistant à mettre en contact du Lipiodol, une molécule ayant une activité anti-tumorale et un hydroxyethylamidon.

19. Procédé de préparation d'une composition selon la revendication 2 **caractérisé en ce qu'**il comprend une étape consistant à mettre en contact du Lipiodol et de l'Idarubicine.

## Patentansprüche

1. Pharmazeutische Zusammensetzung, die Lipiodol, ein Molekül mit einer Antitumoraktivität und eine Hydroxyethylstärke umfasst.

2. Pharmazeutische Zusammensetzung, die Lipiodol und Idarubicin umfasst.

3. Pharmazeutische Zusammensetzung nach Anspruch 1, **dadurch gekennzeichnet, dass** das Molekül mit einer Antitumoraktivität aus der Gruppe umfassend die Antimetabolite, Alkylierungsmittel, Topoisomerasehemmer, interkalierenden Substanzen, Antitumorantibiotika und Mitosespindelgifte ausgewählt ist.

4. Pharmazeutische Zusammensetzung nach Anspruch 3, **dadurch gekennzeichnet, dass** das Antitumormolekül eine interkalierende Substanz, ein Topoisomerasehemmer ist.

5. Pharmazeutische Zusammensetzung nach Anspruch 4, **dadurch gekennzeichnet, dass** die interkalierende Substanz, der Topoisomerasehemmer ein Anthracyclin ist.

6. Pharmazeutische Zusammensetzung nach Anspruch 5, **dadurch gekennzeichnet, dass** das Anthracyclin Idarubicin ist.

7. Pharmazeutische Zusammensetzung nach Anspruch 1, **dadurch gekennzeichnet, dass** die Hydroxyethylstärke ein Molekulargewicht zwischen 100 000 und 250 000 Dalton aufweist.

8. Pharmazeutische Zusammensetzung nach Anspruch 7, **dadurch gekennzeichnet, dass** die Hydroxyethylstärke ein Molekulargewicht zwischen 120 000 und 140 000 Dalton aufweist.

9. Pharmazeutische Zusammensetzung nach Anspruch 1, **dadurch gekennzeichnet, dass** die Hydroxyethylstärke einen molaren Substitutionsgrad zwischen 0,30 und 0,70 aufweist.

10. Pharmazeutische Zusammensetzung nach Anspruch 9, **dadurch gekennzeichnet, dass** die Hydroxyethylstärke einen molaren Substitutionsgrad zwischen 0,35 und 0,45 aufweist.

11. Pharmazeutische Zusammensetzung nach Anspruch 1 oder 2, **dadurch gekennzeichnet, dass** es sich bei dem Lipiodol um Lipiodol Ultra-Fluid handelt.

12. Pharmazeutische Zusammensetzung nach Anspruch 1 oder 3 bis 11, **dadurch gekennzeichnet, dass** die Menge des Moleküls mit einer Antitumoraktivität zwischen 0,004% und 0,2% (Masse/Volumen) in Bezug auf das Gesamtvolumen des Präparats beträgt.

13. Pharmazeutische Zusammensetzung nach Anspruch 2, **dadurch gekennzeichnet, dass** die Menge an Idarubicin zwischen 0,004% und 0,2% (Masse/Volumen) in Bezug auf das Gesamtvolumen des Präparats beträgt.

14. Pharmazeutische Zusammensetzung nach Anspruch 1 oder 3 bis 12, **dadurch gekennzeichnet, dass** die Menge an Hydroxyethylstärke zwischen 0,38% und 2,25% (Masse/Volumen) in Bezug auf das Gesamtvolumen des Präparats beträgt.

15. Pharmazeutische Zusammensetzung nach einem der vorhergehenden Ansprüche, **dadurch gekennzeichnet, dass** die Menge an Lipiodol zwischen 50% und 70% (Masse/Volumen) in Bezug auf das Gesamtvolumen des Präparats beträgt.

16. Pharmazeutische Zusammensetzung nach einem der Ansprüche 1 bis 15 zur Verwendung in der Behandlung von Krebs.

17. Pharmazeutische Zusammensetzung nach einem der Ansprüche 1 bis 15 zur Verwendung in der Behandlung von primärem Leberkrebs.

18. Verfahren zur Herstellung einer Zusammensetzung nach einem der Ansprüche 1, 3 bis 12 oder 14, **dadurch gekennzeichnet, dass** es einen Schritt umfasst, der darin besteht, dass man Lipiodol, ein Molekül mit einer Antitumoraktivität und eine Hydroxyethylstärke miteinander in Kontakt bringt.

19. Verfahren zur Herstellung einer Zusammensetzung nach Anspruch 2, **dadurch gekennzeichnet, dass** es einen Schritt umfasst, der darin besteht, dass man Lipiodol und Idarubicin in Kontakt bringt.

## Claims

1. Pharmaceutical composition comprising Lipiodol and a molecule with anti-tumour activity and a hydroxyethyl starch.

2. Pharmaceutical composition comprising Lipiodol and Idarubicin.

3. Pharmaceutical composition according to claim 1 **characterised in that** said molecule with anti-tumour activity is chosen from the group including antimetabolites, alkylating agents, topoisomerase inhibitors, intercalating agents, anti-tumour antibiotics and mitotic spindle poisons.

4. Pharmaceutical composition according to claim 3 **characterised in that** said anti-tumour molecule is an intercalating agent, topoisomerase inhibitor.

5. Pharmaceutical composition according to claim 4 **characterised in that** said intercalating agent, topoisomerase inhibitor is an anthracycline.

6. Pharmaceutical composition according to claim 5 **characterised in that** said anthracycline is Idarubicin.

7. Pharmaceutical composition according to claim 1 **characterised in that** said hydroxyethyl starch has a molecular weight between 100,000 and 250,000 Dalton.

8. Pharmaceutical composition according to claim 7 **characterised in that** said hydroxyethyl starch has a molecular weight between 120,000 and 140,000 Dalton.

9. Pharmaceutical composition according to claim 1 **characterised in that** said hydroxyethyl starch has a molar substitution ratio between 0.30 and 0.70.

10. Pharmaceutical composition according to claim 9 **characterised in that** said hydroxyethyl starch has a molar substitution ratio between 0.35 and 0.45.

11. Pharmaceutical composition according to claim 1 or 2 **characterised in that** said Lipiodol is Lipiodol Ultra-Fluide.

12. Pharmaceutical composition according to any of claims 1 or 3 to 11 **characterised in that** the quantity of said molecule with anti-tumour activity is between 0.004% and 0.2% (mass/volume) with respect to the total volume of the preparation.

13. Pharmaceutical composition according to claim 2 **characterised in that** the quantity of Idarubicin is between 0.004% and 0.2% (mass/volume) with respect to the total volume of the preparation.

14. Pharmaceutical composition according to any of claims 1 or 3 to 12 **characterised in that** the quantity of hydroxyethyl starch is between 0.38% and 2.25% (mass/volume) with respect to the total volume of the preparation.

15. Pharmaceutical composition according to any of the above claims **characterised in that** the quantity of Lipiodol is between 50% and 70% (mass/volume) with respect to the total volume of the preparation.

16. Pharmaceutical composition according to any of claims 1 to 15 for the use for treating cancer.

17. Pharmaceutical composition according to any of claims 1 to 15 for the use for treating primary liver cancer.

18. Method for preparing a composition according to any of claims 1, 3 to 12 or 14 **characterised in that** it comprises a step consisting of contacting Lipiodol, a molecule with anti-tumour activity and a hydroxyethyl starch.

19. Method for preparing a composition according to claim 2 **characterised in that** it comprises a step consisting of contacting Lipiodol and Idarubicin.
